# EUROPEAN PATENT SPECIFICATION

(11) **EP 0 451 668 B1**
(45) Date of publication and mention of the grant of the patent: **13.08.1997**
(21) Application number: 91105170.4
(22) Date of filing: 02.04.1991
(51) Int. Cl.: C12P 41/00, C12P 7/42

(54) **Process for the production of optically active alkyl 3-aryl-3-hydroxypropionates**
Verfahren zur Herstellung von optisch aktiven Alkyl-3-aryl-3-hydroxypropionaten
Procédé pour la préparation des alkyl-3-aryl-3-hydroxypropionates optiquement actifs

(30) Priority: 12.04.1990 JP 95004/90; 12.04.1990 JP 95005/90
(43) Date of publication of application: 16.10.1991
(73) Proprietor: CHISSO CORPORATION, Osaka-shi Osaka 530 (JP)
(72) Inventor: Miyazawa, Kazutoshi, Ichihara-shi, Chiba-ken (JP); Yoshida, Naoyuki, Ichihara-shi, Chiba-ken (JP)
(74) Representative: Hansen, Bernd, Dr. Dipl.-Chem.

(56) References cited:
- EP-A- 0 321 918
- EP-A- 0 334 966
- US-A- 4 921 798
- JOURNAL OF THE CHEMICAL SOCIETY; vol. 3, 1 February 1985, LETCHWORTH GB, pp.138 - 139; KENSO SOAI ET AL.: 'Asymmetric reduction of prochiral 3-aryl-3- oxoesters with lithium borohyfride using N,N -Dibenzoylcystine as a chiral auxiliary.'
- CHEMICAL ABSTRACTS, vol. 113, no. 3, 16 July 1990, Columbus, Ohio, US; abstract no. 23293, SANTANIELLO, ENZO ET AL.: 'Studies on pig liver esterase-mediated hydrolyses of 3-hydroxy esters' page 600 ;
- TETRAHEDRON LETTERS. vol. 30, no. 15, 1989, OXFORD GB pages 1917 - 1920; YI- FONG WANG ET AL.: 'Lipase-catalyzed irreversible transesterification using enol esters:Resolution of cyanohydrins and syntheses of ethyl-(R)-2-hydroxy-4- phenylbutyrate and (S)-propranolol'
- J. Org. Chem. 1990, 55, p. 812-815

## Description

The present invention relates to methods of producing optically active alkyl 3-aryl-3-hydroxypropionates represented by the general formula: 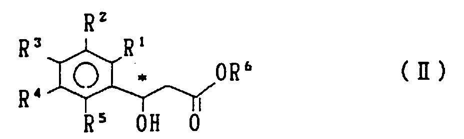 wherein R¹, R², R³, R ⁴ and R⁵ are hydrogen, hydroxyl, alkoxy of 1-4 carbon atoms, benzyloxy, fluorine, chlorine or bromine and R⁶ is alkyl.

The compounds of formula (II) are useful as starting materials for asymmetric synthesis of biologically and pharmacologically active materials.

It is known that in the optically active alkyl 3-aryl-3-hydroxypropionates, ethyl 3-phenyl-3-hydroxypropionate and ethyl 3-(4-methoxyphenyl)-3-hydroxypropionate are obtained by a method of Mukaiyama et al. (Chem. Lett., 813(1985)) in which ketoester is asymmetrically reduced by using diamine, a method of Soul et al. (J. Am. Chem. Soc., 86, 725(1964)) in which a compound is resolved by kinetics with hydrolytic enzyme, and a method of Santaniero et al. (J. Chem. Soc., Perkin Trans. 1, 1987, 2753) in which asymmetric reduction using baker's yeast is mentioned. However, the compounds obtained by these methods have low purity, and the methods are not desirable industrially and effectively.

In other words, in the method of Mukaiyama et al., namely in the asymmetrical reduction of ketoester with diamine, the compound obtained has low purity (44%ee). The reduction is rather impractical because of the reaction conditions at a low temperature, e.g. -100°C.

The advantage of hydrolysis using enzyme is that (-)-and (+)-enantiomers are obtained. However, in the mass production, there are difficult problems such as selection of a reaction solvent, immobilization of enzyme, and purification of the product. The product do not have enough optical purity (about 70%ee).

Moreover, the asymmetric reduction using bread yeast is troublesome and the product has low purity (about 60%ee).

J. Chem. Soc., Chem. Commun, vol. 3, 1985, pp 138-139 discloses that optically active 3-aryl-3-hydroxyesters of high enantiomeric excess can be obtained by the reduction of 3-aryl-3-oxoesters with lithium borohydride which has been chirally modified.

Chem. Abs., vol. 113, no. 3, 1990, no. 23293p discloses that the pig liver esterase-catalysed hydrolysis of PhCH(OH)CH₂CO₂Et proceeds in an aqueous phosphate buffer with moderate enantioselectivity.

EP-A-O 321 918 discloses a method for the enzymatic racemate resolution of a racemic alcohol using a vinyl ester and a lipase. The racemic alcohol to be resolved may include a singly substituted phenyl radical as a substituent.

Tetrahedron Letters, vol. 30, no. 15, 1989, pp 1917-1920 discloses the lipase-catalysed irreversible trans-esterification of an enol ester. The lipase may be derived from a *Pseudomonas* species and the trans-esterification reaction is carried out in the presence of vinyl acetate.

For the above reasons, it is desired to develop a technique for obtaining both enantiomers of optically active alkyl 3-aryl-3-hydroxypropionates useful for common asymmetric synthesis.

The inventors of the present invention carried out research for obtaining efficiently optically active alkyl 3-aryl-3-hydroxypropionates in large quantities. They have found a method for producing in large quantities optically active alkyl 3-aryl-3-hydroxypropionates.

The present invention provides a method for producing an optically active alkyl 3-aryl-3-hydroxypropionate which comprises reacting in the presence of esterase obtainable from a micro-organism or esterase obtainable from an animal, a triglyceride or a fatty acid vinyl ester with an alkyl 3-aryl-3-hydroxypropionate represented by the general formula: 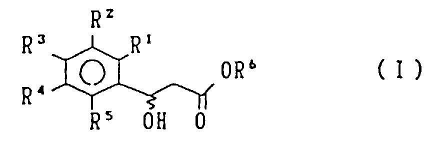 wherein R¹, R², R³, R⁴ and R⁵ are hydrogen, hydroxyl, alkoxy of 1-4 carbon atoms, benzyloxy, fluorine, chlorine or bromine and R⁶ is alkyl, to effect a transesterification reaction, and resolving to an optically active alkyl 3-aryl-3-hydroxypropionate represented by the general formula: 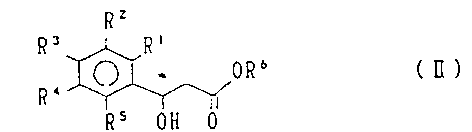 wherein R¹, R², R³, R⁴, R⁵ and R⁶ have the same meanings as described above, and an optically active alkyl 3-aryl-3-acyloxypropionate.

The following compounds are representative optically active alkyl 3-aryl-3-hydroxypropionates (II) which are obtained by the present invention:
(-)-methyl 3-(2-methoxyphenyl)-3-hydroxypropionate,
(-)-ethyl 3-(2-methoxyphenyl)-3-hydroxypropionate,
(-)-propyl 3-(2-methoxyphenyl)-3-hydroxypropionate,
(-)-butyl 3-(2-methoxyphenyl)-3-hydroxypropionate,
(-)-t-butyl 3-(2-methoxyphenyl)-3-hydroxypropionate,
(+)-ethyl 3-(2-methoxyphenyl)-3-hydroxypropionate,
(+)-propyl 3-(2-methoxyphenyl)-3-hydroxypropionate,
(+)-butyl 3-(2-methoxyphenyl)-3-hydroxypropionate,
(+)-t-butyl 3-(2-methoxyphenyl)-3-hydroxypropionate,
(-)-methyl 3-(3-methoxyphenyl)-3-hydroxypropionate,
(-)-ethyl 3-(3-methoxyphenyl)-3-hydroxypropionate,
(-)-propyl 3-(3-methoxyphenyl)-3-hydroxypropionate,
(-)-butyl 3-(3-methoxyphenyl)-3-hydroxypropionate,
(-)-t-butyl 3-(3-methoxyphenyl)-3-hydroxypropionate,
(+)-ethyl 3-(3-methoxyphenyl)-3-hydroxypropionate,
(+)-propyl 3-(3-methoxyphenyl)-3-hydroxypropionate,
(+)-butyl 3-(3-methoxyphenyl)-3-hydroxypropionate,
(+)-t-butyl 3-(3-methoxyphenyl)-3-hydroxypropionate,
(-)-methyl 3-(2,4-dimethoxyphenyl)-3-hydroxypropionate,
(-)-ethyl 3-(2,4-dimethoxyphenyl)-3-hydroxypropionate,
(-)-propyl 3-(2,4-dimethoxyphenyl)-3-hydroxypropionate,
(-)-butyl 3-(2,4-dimethoxyphenyl)-3-hydroxypropionate,
(-)-t-butyl 3-(2,4-dimethoxyphenyl)-3-hydroxypropionate,
(+)-ethyl 3-(2,4-dimethoxyphenyl)-3-hydroxypropionate,
(+)-propyl 3-(2,4-dimethoxyphenyl)-3-hydroxypropionate,
(+)-butyl 3-(2,4-dimethoxyphenyl)-3-hydroxypropionate,
(+)-t-butyl 3-(2,4-dimethoxyphenyl)-3-hydroxypropionate,
(-)-methyl 3-(2,5-dimethoxyphenyl)-3-hydroxypropionate,
(-)-ethyl 3-(2,5-dimethoxyphenyl)-3-hydroxypropionate,
(-)-propyl 3-(2,5-dimethoxyphenyl)-3-hydroxypropionate,
(-)-butyl 3-(2,5-dimethoxyphenyl)-3-hydroxypropionate,
(-)-t-butyl 3-(2,5-dimethoxyphenyl)-3-hydroxypropionate,
(+)-ethyl 3-(2,5-dimethoxyphenyl)-3-hydroxypropionate,
(+)-propyl 3-(2,5-dimethoxyphenyl)-3-hydroxypropionate,
(+)-butyl 3-(2,5-dimethoxyphenyl)-3-hydroxypropionate,
(+)-t-butyl 3-(2,5-dimethoxyphenyl)-3-hydroxypropionate,
(-)-methyl 3-(3,5-dimethoxyphenyl)-3-hydroxypropionate,
(-)-ethyl 3-(3,5-dimethoxyphenyl)-3-hydroxypropionate,
(-)-propyl 3-(3,5-dimethoxyphenyl)-3-hydroxypropionate,
(-)-butyl 3-(3,5-dimethoxyphenyl)-3-hydroxypropionate,
(-)-t-butyl 3-(3,5-dimethoxyphenyl)-3-hydroxypropionate,
(+)-ethyl 3-(3,5-dimethoxyphenyl)-3-hydroxypropionate,
(+)-propyl 3-(3,5-dimethoxyphenyl)-3-hydroxypropionate,
(+)-butyl 3-(3,5-dimethoxyphenyl)-3-hydroxypropionate,
(+)-t-butyl 3-(3,5-dimethoxyphenyl)-3-hydroxypropionate,
(-)-methyl 3-(3,4-dimethoxyphenyl)-3-hydroxypropionate,
(-)-ethyl 3-(3,4-dimethoxyphenyl)-3-hydroxypropionate,
(-)-propyl 3-(3,4-dimethoxyphenyl)-3-hydroxypropionate,
(-)-butyl 3-(3,4-dimethoxyphenyl)-3-hydroxypropionate,
(-)-t-butyl 3-(3,4-dimethoxyphenyl)-3-hydroxypropionate,
(+)-ethyl 3-(3,4-dimethoxyphenyl)-3-hydroxypropionate,
(+)-propyl 3-(3,4-dimethoxyphenyl)-3-hydroxypropionate,
(+)-butyl 3-(3,4-dinethoxyphenyl)-3-hydroxypropionate,
(+)-t-butyl 3-(3,4-dimethoxyphenyl)-3-hydroxypropionate,
(-)-methyl 3-(2-benzyloxyphenyl)-3-hydroxypropionate,
(-)-ethyl 3-(2-benzyloxyphenyl)-3-hydroxypropionate,
(-)-propyl 3-(2-benzyloxyphenyl)-3-hydroxypropionate,
(-)-butyl 3-(2-benzyloxyphenyl)-3-hydroxypropionate,
(-)-t-butyl 3-(2-benzyloxyphenyl)-3-hydroxypropionate,
(+)-methyl 3-(2-benzyloxyphenyl)-3-hydroxypropionate,
(+)-ethyl 3-(2-benzyloxyphenyl)-3-hydroxypropionate,
(+)-propyl 3-(2-benzyloxyphenyl)-3-hydroxypropionate,
(+)-butyl 3-(2-benzyloxyphenyl)-3-hydroxypropionate,
(+)-t-butyl 3-(2-benzyloxyphenyl)-3-hydroxypropionate,
(-)-methyl 3-(3-benzyloxyphenyl)-3-hydroxypropionate,
(-)-ethyl 3-(3-benzyloxyphenyl)-3-hydroxypropionate,
(-)-propyl 3-(3-benzyloxyphenyl)-3-hydroxypropionate,
(-)-butyl 3-(3-benzyloxyphenyl)-3-hydroxypropionate,
(-)-t-butyl 3-(3-benzyloxyphenyl)-3-hydroxypropionate,
(+)-methyl 3-(3-benzyloxyphenyl)-3-hydroxypropionate,
(+)-ethyl 3-(3-benzyloxyphenyl)-3-hydroxypropionate,
(+)-propyl 3-(3-benzyloxyphenyl)-3-hydroxypropionate,
(+)-butyl 3-(3-benzyloxyphenyl)-3-hydroxypropionate,
(+)-t-butyl 3-(3-benzyloxyphenyl)-3-hydroxypropionate,
(-)-methyl 3-(4-benzyloxyphenyl)-3-hydroxypropionate,
(-)-ethyl 3-(4-benzyloxyphenyl)-3-hydroxypropionate,
(-)-propyl 3-(4-benzyloxyphenyl)-3-hydroxypropionate,
(-)-butyl 3-(4-benzyloxyphenyl)-3-hydroxypropionate,
(-)-t-butyl 3-(4-benzyloxyphenyl)-3-hydroxypropionate,
(+)-methyl 3-(4-benzyloxyphenyl)-3-hydroxypropionate,
(+)-ethyl 3-(4-benzyloxyphenyl)-3-hydroxypropionate,
(+)-propyl 3-(4-benzyloxyphenyl)-3-hydroxypropionate,
(+)-butyl 3-(4-benzyloxyphenyl)-3-hydroxypropionate,
(+)-t-butyl 3-(4-benzyloxyphenyl)-3-hydroxypropionate,
(-)-methyl 3-(2-fluorophenyl)-3-hydroxypropionate,
(-)-ethyl 3-(2-fluorophenyl)-3-hydroxypropionate,
(-)-propyl 3-(2-fluorophenyl)-3-hydroxypropionate,
(-)-butyl 3-(2-fluorophenyl)-3-hydroxypropionate,
(-)-t-butyl 3-(2-fluorophenyl)-3-hydroxypropionate,
(+)-methyl 3-(2-fluorophenyl)-3-hydroxypropionate,
(+)-ethyl 3-(2-fluorophenyl)-3-hydroxypropionate,
(+)-propyl 3-(2-fluorophenyl)-3-hydroxypropionate,
(+)-butyl 3-(2-fluorophenyl)-3-hydroxypropionate,
(+)-t-butyl 3-(2-fluorophenyl)-3-hydroxypropionate,
(-)-methyl 3-(3-fluorophenyl)-3-hydroxypropionate,
(-)-ethyl 3-(3-fluorophenyl)-3-hydroxypropionate,
(-)-propyl 3-(3-fluorophenyl)-3-hydroxypropionate,
(-)-butyl 3-(3-fluorophenyl)-3-hydroxypropionate,
(-)-t-butyl 3-(3-fluorophenyl)-3-hydroxypropionate,
(+)-methyl 3-(4-fluorophenyl)-3-hydroxypropionate,
(+)-ethyl 3-(4-fluorophenyl)-3-hydroxypropionate,
(+)-propyl 3-(4-fluorophenyl)-3-hydroxypropionate,
(+)-butyl 3-(4-fluorophenyl)-3-hydroxypropionate,
(+)-t-butyl 3-(4-fluorophenyl)-3-hydroxypropionate,
(-)-methyl 3-(2,3-difluorophenyl)-3-hydroxypropionate,
(-)-ethyl 3-(2,3-difluorophenyl)-3-hydroxypropionate,
(-)-propyl 3-(2,3-difluorophenyl)-3-hydroxypropionate,
(-)-butyl 3-(2,3-difluorophenyl)-3-hydroxypropionate,
(-)-t-butyl 3-(2,3-difluorophenyl)-3-hydroxypropionate,
(+)-methyl 3-(2,3-difluorophenyl)-3-hydroxypropionate,
(+)-ethyl 3-(2,3-difluorophenyl)-3-hydroxypropionate,
(+)-propyl 3-(2,3-difluorophenyl)-3-hydroxypropionate,
(+)-butyl 3-(2,3-difluorophenyl)-3-hydroxypropionate,
(+)-t-butyl 3-(2,3-difluorophenyl)-3-hydroxypropionate,
(-)-methyl 3-(3,4-difluorophenyl)-3-hydroxypropionate,
(-)-ethyl 3-(3,4-difluorophenyl)-3-hydroxypropionate,
(-)-propyl 3-(3,4-difluorophenyl)-3-hydroxypropionate,
(-)-butyl 3-(3,4-difluorophenyl)-3-hydroxypropionate,
(-)-t-butyl 3-(3,4-difluorophenyl)-3-hydroxypropionate,
(+)-methyl 3-(3,4-difluorophenyl)-3-hydroxypropionate,
(+)-ethyl 3-(3,4-difluorophenyl)-3-hydroxypropionate,
(+)-propyl 3-(3,4-difluorophenyl)-3-hydroxypropionate,
(+)-butyl 3-(3,4-difluorophenyl)-3-hydroxypropionate,
(+)-t-butyl 3-(3,4-difluorophenyl)-3-hydroxypropionate,
(-)-methyl 3-(3,5-difluorophenyl)-3-hydroxypropionate,
(-)-ethyl 3-(3,5-difluorophenyl)-3-hydroxypropionate,
(-)-propyl 3-(3,5-difluorophenyl)-3-hydroxypropionate,
(-)-butyl 3-(3,5-difluorophenyl)-3-hydroxypropionate,
(-)-t-butyl 3-(3,5-difluorophenyl)-3-hydroxypropionate,
(+)-methyl 3-(3,5-difluorophenyl)-3-hydroxypropionate,
(+)-ethyl 3-(3,5-difluorophenyl)-3-hydroxypropionate,
(+)-propyl 3-(3,5-difluorophenyl)-3-hydroxypropionate,
(+)-butyl 3-(3,5-difluorophenyl)-3-hydroxypropionate,
(+)-t-butyl 3-(3,5-difluorophenyl)-3-hydroxypropionate,
(-)-methyl 3-(2-chlorophenyl)-3-hydroxypropionate,
(-)-ethyl 3-(2-chlorophenyl)-3-hydroxypropionate,
(-)-propyl 3-(2-chlorophenyl)-3-hydroxypropionate,
(-)-butyl 3-(2-chlorophenyl)-3-hydroxypropionate,
(-)-t-butyl 3-(2-chlorophenyl)-3-hydroxypropionate,
(+)-methyl 3-(2-chlorophenyl)-3-hydroxypropionate,
(+)-ethyl 3-(2-chlorophenyl)-3-hydroxypropionate,
(+)-propyl 3-(2-chlorophenyl)-3-hydroxypropionate,
(+)-butyl 3-(2-chlorophenyl)-3-hydroxypropionate,
(+)-t-butyl 3-(2-chlorophenyl)-3-hydroxypropionate,
(-)-methyl 3-(3-chlorophenyl)-3-hydroxypropionate,
(-)-ethyl 3-(3-chlorophenyl)-3-hydroxypropionate,
(-)-propyl 3-(3-chlorophenyl)-3-hydroxypropionate,
(-)-butyl 3-(3-chlorophenyl)-3-hydroxypropionate,
(-)-t-butyl 3-(3-chlorophenyl)-3-hydroxypropionate,
(+)-methyl 3-(3-chlorophenyl)-3-hydroxypropionate,
(+)-ethyl 3-(3-chlorophenyl)-3-hydroxypropionate,
(+)-propyl 3-(3-chlorophenyl)-3-hydroxypropionate,
(+)-butyl 3-(3-chlorophenyl)-3-hydroxypropionate,
(+)-t-butyl 3-(3-chlorophenyl)-3-hydroxypropionate,
(-)-methyl 3-(4-chlorophenyl)-3-hydroxypropionate,
(-)-ethyl 3-(4-chlorophenyl)-3-hydroxypropionate,
(-)-propyl 3-(4-chlorophenyl)-3-hydroxypropionate,
(-)-butyl 3-(4-chlorophenyl)-3-hydroxypropionate,
(-)-t-butyl 3-(4-chlorophenyl)-3-hydroxypropionate,
(+)-methyl 3-(4-chlorophenyl)-3-hydroxypropionate,
(+)-ethyl 3-(4-chlorophenyl)-3-hydroxypropionate,
(+)-propyl 3-(4-chlorophenyl)-3-hydroxypropionate,
(+)-butyl 3-(4-chlorophenyl)-3-hydroxypropionate,
(+)-t-butyl 3-(4-chlorophenyl)-3-hydroxypropionate,
(-)-methyl 3-(2,3-dichlorophenyl)-3-hydroxypropionate,
(-)-ethyl 3-(2,3-dichlorophenyl)-3-hydroxypropionate,
(-)-propyl 3-(2,3-dichlorophenyl)-3-hydroxypropionate,
(-)-butyl 3-(2,3-dichlorophenyl)-3-hydroxypropionate,
(-)-t-butyl 3-(2,3-dichlorophenyl)-3-hydroxypropionate,
(+)-methyl 3-(2,3-dichlorophenyl)-3-hydroxypropionate,
(+)-ethyl 3-(2,3-dichlorophenyl)-3-hydroxypropionate,
(+)-propyl 3-(2,3-dichlorophenyl)-3-hydroxypropionate,
(+)-butyl 3-(2,3-dichlorophenyl)-3-hydroxypropionate,
(+)-t-butyl 3-(2,3-dichlorophenyl)-3-hydroxypropionate,
(-)-methyl 3-(3,4-dichlorophenyl)-3-hydroxypropionate,
(-)-ethyl 3-(3,4-dichlorophenyl)-3-hydroxypropionate,
(-)-propyl 3-(3,4-dichlorophenyl)-3-hydroxypropionate,
(-)-butyl 3-(3,4-dichlorophenyl)-3-hydroxypropionate,
(-)-t-butyl 3-(3,4-dichlorophenyl)-3-hydroxypropionate,
(+)-methyl 3-(3,4-dichlorophenyl)-3-hydroxypropionate,
(+)-ethyl 3-(3,4-dichlorophenyl)-3-hydroxypropionate,
(+)-propyl 3-(3,4-dichlorophenyl)-3-hydroxypropionate,
(+)-butyl 3-(3,4-dichlorophenyl)-3-hydroxypropionate,
(+)-t-butyl 3-(3,4-dichlorophenyl)-3-hydroxypropionate,
(-)-methyl 3-(2-bromophenyl)-3-hydroxypropionate,
(-)-ethyl 3-(2-bromophenyl)-3-hydroxypropionate,
(-)-propyl 3-(2-bromophenyl)-3-hydroxypropionate,
(-)-butyl 3-(2-bromophenyl)-3-hydroxypropionate,
(-)-t-butyl 3-(2-bromophenyl)-3-hydroxypropionate,
(+)-methyl 3-(2-bromophenyl)-3-hydroxypropionate,
(+)-ethyl 3-(2-bromophenyl)-3-hydroxypropionate,
(+)-propyl 3-(2-bromophenyl)-3-hydroxypropionate,
(+)-butyl 3-(2-bromophenyl)-3-hydroxypropionate,
(-)-methyl 3-(3-bromophenyl)-3-hydroxypropionate,
(-)-ethyl 3-(3-bromophenyl)-3-hydroxypropionate,
(-)-propyl 3-(3-bromophenyl)-3-hydroxypropionate,
(-)-butyl 3-(3-bromophenyl)-3-hydroxypropionate,
(-)-t-butyl 3-(3-bromophenyl)-3-hydroxypropionate,
(+)-methyl 3-(3-bromophenyl)-3-hydroxypropionate,
(+)-ethyl 3-(3-bromophenyl)-3-hydroxypropionate,
(+)-propyl 3-(3-bromophenyl)-3-hydroxypropionate,
(+)-butyl 3-(3-bromophenyl)-3-hydroxypropionate,
(+)-t-butyl 3-(3-bromophenyl)-3-hydroxypropionate,
(-)-methyl 3-(4-bromophenyl)-3-hydroxypropionate,
(-)-ethyl 3-(4-bromophenyl)-3-hydroxypropionate,
(-)-propyl 3-(4-bromophenyl)-3-hydroxypropionate,
(-)-butyl 3-(4-bromophenyl)-3-hydroxypropionate,
(-)-t-butyl 3-(4-bromophenyl)-3-hydroxypropionate,
(+)-methyl 3-(4-bromophenyl)-3-hydroxypropionate,
(+)-ethyl 3-(4-bromophenyl)-3-hydroxypropionate,
(+)-propyl 3-(4-bromophenyl)-3-hydroxypropionate,
(+)-butyl 3-(4-bromophenyl)-3-hydroxypropionate,
(+)-t-butyl 3-(4-bromophenyl)-3-hydroxypropionate,
(-)-methyl 3-(2,3-dibromophenyl)-3-hydroxypropionate,
(-)-ethyl 3-(2,3-dibromophenyl)-3-hydroxypropionate,
(-)-propyl 3-(2,3-dibromophenyl)-3-hydroxypropionate,
(-)-butyl 3-(2,3-dibromophenyl)-3-hydroxypropionate,
(-)-t-butyl 3-(2,3-dibromophenyl)-3-hydroxypropionate,
(+)-methyl 3-(2,3-dibromophenyl)-3-hydroxypropionate,
(+)-ethyl 3-(2,3-dibromophenyl)-3-hydroxypropionate,
(+)-propyl 3-(2,3-dibromophenyl)-3-hydroxypropionate,
(+)-butyl 3-(2,3-dibromophenyl)-3-hydroxypropionate,
(+)-t-butyl 3-(2,3-dibromophenyl)-3-hydroxypropionate,
(-)-methyl 3-(3,4-dibromophenyl)-3-hydroxypropionate,
(-)-ethyl 3-(3,4-dibromophenyl)-3-hydroxypropionate,
(-)-propyl 3-(3,4-dibromophenyl)-3-hydroxypropionate,
(-)-butyl 3-(3,4-dibromophenyl)-3-hydroxypropionate,
(-)-t-butyl 3-(3,4-dibromophenyl)-3-hydroxypropionate,
(+)-methyl 3-(3,4-dibromophenyl)-3-hydroxypropionate,
(+)-ethyl 3-(3,4-dibromophenyl)-3-hydroxypropionate,
(+)-propyl 3-(3,4-dibromophenyl)-3-hydroxypropionate,
(+)-butyl 3-(3,4-dibromophenyl)-3-hydroxypropionate,
(+)-t-butyl 3-(3,4-dibromophenyl)-3-hydroxypropionate,
(-)-methyl 3-(3,5-dibromophenyl)-3-hydroxypropionate,
(-)-ethyl 3-(3,5-dibromophenyl)-3-hydroxypropionate,
(-)-propyl 3-(3,5-dibromophenyl)-3-hydroxypropionate,
(-)-butyl 3-(3,5-dibromophenyl)-3-hydroxypropionate,
(-)-t-butyl 3-(3,5-dibromophenyl)-3-hydroxypropionate,
(+)-methyl 3-(3,5-dibromophenyl)-3-hydroxypropionate,
(+)-ethyl 3-(3,5-dibromophenyl)-3-hydroxypropionate,
(+)-propyl 3-(3,5-dibromophenyl)-3-hydroxypropionate,
(+)-butyl 3-(3,5-dibromophenyl)-3-hydroxypropionate,
(+)-t-butyl 3-(3,5-dibromophenyl)-3-hydroxypropionate,
(-)-methyl 3-(3,5-dichlorophenyl)-3-hydroxypropionate,
(-)-ethyl3-(3,5-dichlorophenyl)-3-hydroxypropionate,
(-)-propyl 3-(3,5-dichlorophenyl)-3-hydroxypropionate,
(-)-butyl 3-(3,5-dichlorophenyl)-3-hydroxypropionate,
(-)-t-butyl 3-(3,5-dichlorophenyl)-3-hydroxypropionate,
(+)-methyl 3-(3,5-dichlorophenyl)-3-hydroxypropionate,
(+)-ethyl 3-(3,5-dichlorophenyl)-3-hydroxypropionate,
(+)-propyl 3-(3,5-dichlorophenyl)-3-hydroxypropionate,
(+)-butyl 3-(3,5-dichlorophenyl)-3-hydroxypropionate,
(+)-t-butyl 3-(3,5-dichlorophenyl)-3-hydroxypropionate and the like.

In this invention, the alkyl 3-aryl-3-hydroxypropionates of the raw materials are easily obtained by reacting an aryl aldehydes with ethyl bromoacetate in the presence of zinc. They are also obtained by the reduction of appropriate arylcarbonylacetate esters.

As for triglycerides in the enzyme reaction, triacetin, tripropionin, tributyrin, tricaproin, trilaurin, etc. can be exemplified, and as for fatty acid vinyl esters, vinyl acetate, vinyl propionate, vinyl butyrate, vinyl caproate, vinyl laurate, etc. can be exemplified. These compounds are commercially available and also easily obtained by synthesis.

The following table shows commercially available esterases that can be used in the present invention.

**Table**

| Trade name | Origin | Seller or Maker |
|---|---|---|
| Lipase PS | Pseudomonas fluorescens | Amano Pharmaceutical Co., Ltd |
| Lipase CES | Pseudomonas sp. | " |
| Lipase AP | Aspergillus niger | " |
| Lipase M | Mucor javanicus | " |
| Lipase CE | Humicola lanuginosa | " |
| Lipase F-AP | Rhizopus javanicus | " |
| Lipase II | Porcine pancreas | Sigma Chemical |
| Lipase VIII | Geotrichum candidum | " |
| Lipase X | Rhizopus delamar | " |
| Lipase | Chromobacterium viscosum | Toyo Jozo Co., Ltd |
| Lipase A | Aspergillus niger | Novo Industi A/S |
| Lipase | Rhizopus niveus | Nagase Biochemicals, Co. Ltd |
| Lipase B | Pseudomonas fragi | Sapporo Beer Co., Ltd |

In addition to these enzymes, microorganisms which produce the enzymes having the above ability can be used regardless of their species and genus. As such microorganisms, the genera Pseudomonas, Arthrobacter, Acromobacter, Alcaligenes, Asperigillus, Chromobacterium, Candida, Mucor, Rhizopus, etc, can be exemplified.

In these microorganisms, the genus Pseudomonas is more preferable.

The reaction is conducted by mixing an alkyl 3-aryl-3-hydroxypropionate with a fatty acid vinyl ester or a triglyceride, and contacting efficiently the mixture with an enzyme. The reaction temperature is suitably room temperature (about 10°C) to 150°C, preferably 20° to 45°C. The reaction time is widely variable, say 1 to 1000 hours.

The alkyl 3-aryl-3-hydroxypropionate which is a substrate and the fatty acid vinyl ester or the triglyceride are mixed in the ratio 1:0.5 to 1:10 by mole, and preferably 1:0.5 by mole.

As for a reaction solvent which does not inhibit esterase activities, if necessary, hydrocarbons such as n-hexane, n-heptane, etc., benzene, toluene, ethers and the like can be used. Otherwise, there is no necessity of such solvents.

After the enzyme reaction as described above, the esterase can be removed by conventional filter operation and used again, as it is. The reactant which is the filtrate can be separated into an optically active alkyl 3-aryl-3-hydroxypropionate and an optically active alkyl 3-aryl-3-acyloxypropionate, respectively, for instance by distillation under reduced pressure or column chromatography. After the reaction, when the optical purity of the optically active alkyl 3-aryl-3-hydroxypropionate is low (when the transesterification is not enough), the compound having high optical purity (>99%ee) can be obtained by re-transesterification with the esterase.

The optically active alkyl 3-aryl-3-acyloxypropionates obtained by the above processes are hydrolyzed in an alkali (e. g. potassium hydroxide or sodium hydroxide) or an acid (e. g. hydrochloric acid or sulfuric acid) or alcoholized (in methanol or ethanol) to derive antipodes of the above optically active alkyl 3-aryl-3-hydroxypropionates or optically active 3-aryl-3-hydroxy propionic acid.

By the above processes, the optically active (+)- and (-)-alkyl 3-aryl-3-hydroxypropionates can be obtained. Further, (+)- or (-)-alkyl 3-aryl-3-acyloxypropionates are dependent on the esterases used.

The method of the present invention is excellent in the efficient mass production of optically active alkyl 3-aryl-3-hydroxypropionates having high optical purities.

The optically active alkyl 3-aryl-3-hydroxypropionates are useful as starting materials for asymmetric synthesis in wide application.

For example, optically active ethyl 3-phenyl-3-hydroxypropionate which is a known compound is reduced to obtain optically active 1-phenyl-1,3-propanediol. The compound obtained is a starting material of fluoxetin which is an antidepressant (J. Am. Chem. Soc., 53, 4081(1988)). Moreover, the compounds obtained by the method of the present invention can be lead to various kinds of materials having biological and pharmacological activity.

The following examples illustrate the present invention more specifically.

### Example 1

The mixture of 5.0 g (25.7 mmol) of ethyl 3-phenyl-3-hydroxypropionate, 1.83 g (13 mmol) of vinyl caproate and 1.0 g of lipase PS was stirred at room temperature for 35 hours. After the lipase was removed by filtration, the filtrate was eluted by column chromatography (eluting solution; 9/1 of toluene/ethyl acetate) to obtain 2.2 g (>99%ee) of (-)-ethyl 3-phenyl-3-hydroxypropionate and 4.1 g (about 90%ee) of (+)-ethyl 3-phenyl-3-hexanoyloxypropionate.

To determine the optical purity, (+)-ethyl 3-phenyl-3-hydroxypropionate which was obtained by alcoholysis of (+)-ethyl 3-phenyl-3-hexanoyloxypropionate in ethanol was used.

The optical purity was determined by the following method: after the optically active compounds were reacted with 4-nitrophenyl isocyanate, the compounds obtained were eluted by HPLC (Japanese Patent Application No. 1-176580).

### Example 2

The mixture of 4.0 g (21 mmol) of S-ethyl 3-phenyl-3-hydroxypropionate having a low optical purity of 13.3%ee, 2.2 g (15 mmol) of vinyl caproate and 2.0 g of lipase PS was stirred at room temperature for 30 hours. After the lipase was removed by filtration, the filtrate was eluted by column chromatography (eluting solution; 9/1 of toluene/ethyl acetate). 1.7 g (99%ee) of (-)-ethyl 3-phenyl-3-hydroxypropionate having high optical purity and 2.7 g of (+)-ethyl 3-phenyl-3-hexanoyloxy propionate were obtained, respectively.

### Example 3

The mixture of 5.0 g (28 mmol) of methyl 3-phenyl-3-hydroxypropionate, 1.97 g (14 mmol) of vinyl caproate and 2.0 g of lipase PS was stirred at room temperature for 28 hours. After the lipase was removed by filtration, the filtrate was eluted by column chromatography (eluting solution; 9/1 of toluene/ethyl acetate). 2.35 g (88%ee) of (-)-methyl 3-phenyl-3-hydroxypropionate and 3.97 g (91%ee) of (+)-methyl 3-phenyl-3-hexanoyloxy-propionate were obtained, respectively.

The optical purity of (+)-methyl 3-phenyl-3-hexanoyloxypropionate was determined by using (+)-ethyl 3-phenyl-3-hydroxypropionate obtained by alcoholysis of the former compound in ethanol.

The optical purities of both (+)- and (-)- compounds were determined by the same method as described in Example 1.

### Example 4

The mixture of 5.0 g of ethyl 3-(4-methoxyphenyl)-3-hydroxypropionate, 1.6 g of vinyl caproate and 2.0 g of lipase PS was stirred at room temperature for 30 hours. After the lipase was removed by filtration, the filtrate was eluted by column chromatography (eluting solution; 3/1 of toluene/ethyl acetate). 3.8 g of (-)-ethyl 3-(4-methoxyphenyl)-3-hydroxypropionate was obtained. The specific rotation of the compound was [α]_{D} -19°(c1.22, CHCl₃).

### Example 5

The mixture of 5.0 g (22 mmol) of ethyl 3-(2-methoxyphenyl)-3-hydroxypropionate, 1.6 g (11 mmol) of vinyl caproate and 2.0 g of lipase PS was stirred at room temperature for 3 days. After the lipase was removed by filtration, the filtrate was eluted by column chromatography (eluting solution; 5/1 of toluene/ethyl acetate). 1.9 g (45%ee) of (-)-ethyl 3-(2-methoxyphenyl)-3-hydroxypropionate was obtained. The specific rotation of the compound was [α]_{D}³¹ -27.1°(c1.05, CHCl₃).

Further, 5.4 g of (+)-ethyl 3-(2-methoxyphenyl)-3-hexanoyloxypropionate was obtained. The specific rotation of the compound was [α]_{D}³⁰ +32.0°(c0.20, CHCl₃).

To determine optical purity of (+)-ethyl 3-(2-methoxyphenyl)-3-hexanoyloxypropionate, the compound was converted into (+)-3-(2-methoxyphenyl)-1,3-propanediol with lithium aluminium hydride, and the compound obtained was analyzed with an optical resolution HPLC (Chiral Cel OB, manufactured by Daisel Co.). The result was 74%ee.

### Example 6

The mixture of 5.0 g (20 mmol) of ethyl 3-(3,4-dimethoxyphenyl)-3-hydroxypropionate, 1.4 g (13 mmol) of vinyl caproate and 1.0 g of lipase PS was stirred at room temperature for 12 days. After the lipase was removed by filtration, the filtrate was eluted by column chromatography (eluting solution; 9/1 of toluene/ethyl acetate) to obtain 2 g (>50%ee) of (-)-ethyl 3-(3,4-dimethoxyphenyl)-3-hydroxypropionate and 4.3 g (about 43%ee) of (+)-ethyl 3-(3,4-dimethoxyphenyl)-3-hexanoyloxypropionate.

The optical purity was determined by the following method: after (-)-ethyl 3-(3,4-dimethoxyphenyl)-3-hydroxypropionate was reacted with 4-nitrophenyl isocyanate, the compounds obtained were eluted by HPLC (Japanese Patent Application No. 1-176580). The specific rotation of the compound was [α]_{D}²¹ -16.9°(c1.0, CDCl₃).

To determine optical purity of (+)-ethyl 3-(3,4-dimethoxyphenyl)-3-hexanoyloxypropionate, the compound was converted into (+)-3-(3,4-dimethoxyphenyl)-1,3-propanediol with lithium aluminium hydride, and the compound obtained was analyzed with an optical resolution HPLC (Chiral Cel OB, manufactured by Daisel Co.).

### Example 7

The mixture of 5.0 g (20 mmol) of ethyl 3-(2,4-dimethoxyphenyl)-3-hydroxypropionate, 10 ml of vinyl acetate and 2.0 g of lipase PS was stirred at room temperature for 50 days. After the lipase was removed by filtration, the filtrate was eluted by column chromatography (eluting solution; 9/1 of toluene/ethyl acetate) to obtain 3.5 g (35%ee) of (-)-ethyl 3-(2,4-dimethoxyphenyl)-3-hydroxypropionate and 1.5 g of (+)-ethyl 3-(2,4-dimethoxyphenyl)-3-acetoxypropionate, respectively.

The optical purity was determined by the following method: after (-)-ethyl 3-(2,4-dimethoxyphenyl)-3-hydroxypropionate was reacted with 4-nitrophenyl isocyanate, the compounds obtained were eluted by HPLC. The specific rotation of the compound was [α]_{D}²¹ -12.4°(c1.1, CDCl₃).

### Example 8

The mixture of 5.0 g (20 mmol) of ethyl 3-(3,5-dimethoxyphenyl)-3-hydroxypropionate, 1.4 g (16 mmol) of vinyl acetate and 2.0 g of lipase PS was stirred at room temperature for 8 days. After the lipase was removed by filtration, the filtrate was eluted by column chromatography (eluting solution; 9/1 of toluene/ethyl acetate) to obtain 2.2 g (50%ee) of (-)-ethyl 3-(3,5-dimethoxyphenyl)-3-hydroxypropionate and 2.7 g of (+)-ethyl 3-(3,5-di-methoxyphenyl)-3-acetoxypropionate.

The optical purity was determined by the following method: after (-)-ethyl 3-(3,4-dimethoxyphenyl)-3-hydroxypropionate was reacted with 4-nitrophenyl isocyanate, the compounds obtained were eluted by HPLC. The specific rotation of the compound was [α]_{D}²⁴ -11.7°(c1.1, CDCl₃).

### Example 9

The mixture of 5.0 g of ethyl 3-(3-chlorophenyl)-3-hydroxypropionate, 1.6 g of vinyl caproate and 2.0 g of lipase PS was stirred at room temperature for 13 days. After the lipase was removed by filtration, the filtrate was eluted by column chromatography (eluting solution; 9/1 of toluene/ethyl acetate) to obtain 2.34 g (40%ee) of (-)-ethyl 3-(3-chlorophenyl)-3-hydroxypropionate and 2.75 g (>90%ee) of (+)-ethyl 3-(3-chlorophenyl)-3-caproyloxypropionate.

The optical purity was determined by the following method: after (-)-ethyl 3-(3,4-dimethoxyphenyl)-3-hydroxypropionate was reacted with 4-nitrophenyl isocyanate, the compounds obtained were eluted by HPLC. To determin the optical purity of the other compound, (+)-ethyl 3-(3-chlorophenyl)-3-acetyloxypropionate, the compound was converted into (+)-3-(3-chlorophenyl) 1,3-propanediol with lithium aluminium hydride, and the compound obtained was analyzed with an optical resolution HPLC (Chiral Cel OB, manufactured by Daisel Co.). The specific rotation of the compound was [α]_{D} -16.4°(c1.0, CDCl₃).

### Example 10

The mixture of 5.0 g of ethyl 3-(4-chlorophenyl)-3-hydroxypropionate, 1.6 g of vinyl caproate and 2.0 g of lipase PS was stirred at room temperature for 13 days. After the lipase was removed by filtration, the filtrate was eluted by column chromatography (eluting solution; 9/1 of toluene/ethyl acetate) to obtain 2.1 g of (-)-ethyl 3-(4-chlorophenyl)-3-hydroxypropionate and 2.7 g of (+)-ethyl 3-(4-chlorophenyl)-3-caproyloxypropionate, respectively.

The specific rotation of (-)-ethyl 3-(4-chlorophenyl) -3-hydroxypropionate was [α]_{D} -20.2°(c1.0, CDCl₃) and that of (+)-ethyl 3(4-chlorophenyl)-3-caproyloxypropionate was [α]_{D} -11.0°(c1.0, CDCl₃).

## Claims

1. A method for producing an optically active alkyl 3-aryl-3-hydroxypropionate, characterized in that the method comprises reacting, in the presence of esterase obtainable from a micro-organism or esterase obtainable from an animal, a triglyceride with an alkyl 3-aryl-3-hydroxypropionate represented by the general formula: 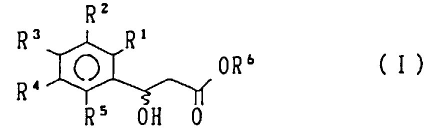 wherein R¹, R², R³, R⁴ and R⁵ are hydrogen, hydroxyl, alkoxy of 1-4 carbon atoms, benzyloxy, fluorine, chlorine, or bromine and R⁶ is alkyl,
to effect a transesterification reaction, and resolving to an optically active alkyl 3-aryl-3-hydroxypropionate represented by the general formula: 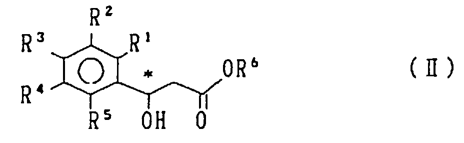 wherein R¹, R², R³, R⁴, R⁵ and R⁶ have the same meanings as described above,
and an optically active alkyl 3-aryl-3-acyloxy propionate.

2. A method for producing an optically active alkyl 3-aryl-3-hydroxypropionate, characterized in that the method comprises reacting in the presence of esterase obtainable from a micro-organism or esterase obtainable from an animal, a fatty acid vinyl ester and an alkyl 3-aryl-3-hydroxypropionate represented by the general formula: 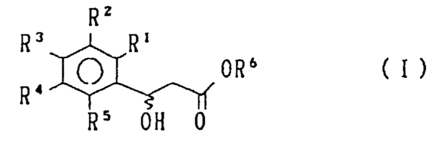 wherein R¹, R², R³, R⁴ and R⁵ are hydrogen, hydroxyl, alkoxy of 1-4 carbon atoms, benzyloxy, fluorine, chlorine, or bromine and R⁶ is alkyl,
to effect a transesterification reaction, and resolving to an optically active alkyl 3-aryl-3-hydroxypropionate represented by the general formula: 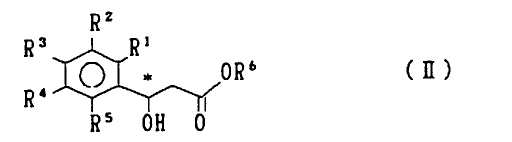 wherein R¹, R², R³, R⁴, R⁵ and R⁶ have the same meanings as described above,
and an optically active alkyl 3-aryl-3-acyloxy propionate.

## Patentansprüche

1. Verfahren zur Erzeugung eines optisch aktiven Alkyl-3-aryl-3-hydroxypropionats, dadurch gekennzeichnet, daß das Verfahren die Reaktion eines Triglycerides mit einem Alkyl-3-aryl-3-hydroxypropionat, dargestellt durch die allgemeine Formel (I), in der Gegenwart von Esterase, erhältlich von einem Mikroorganismus, oder Esterase, erhältlich von einem Tier: 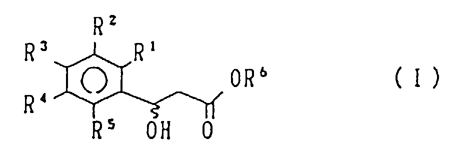 worin R¹, R², R³, R⁴ und R⁵ Wasserstoff, Hydroxyl, Alkoxy mit 1 bis 4 Kohlenstoffatomen, Benzyloxy, Fluor, Chlor oder Brom sind und R⁶ Alkyl ist, zur Bewirkung einer Umesterungsreaktion, und Auflösen in ein optisch aktives Alkyl-3-aryl-3-hydroxypropionat, dargestellt durch die allgemeine Formel: 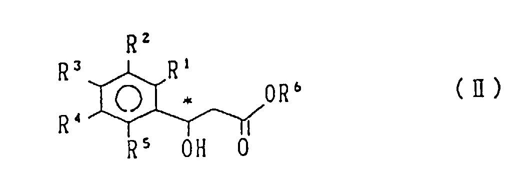 worin R¹, R², R³, R⁴, R⁵ und R⁶ die oben beschriebenen Bedeutungen haben, und ein optisch aktives Alkyl-3-acyloxypropionat umfaßt.

2. Verfahren zur Erzeugung eines optisch aktiven Alkyl-3-aryl-3-hydroxypropionates, dadurch gekennzeichnet, daß das Verfahren die Reaktion eines Fettsäurevinylesters und eines Alkyl-3-aryl-3-hydroxypropionates, dargestellt durch die allgemeine Formel (I) in der Gegenwart von Esterase, erhältlich von einem Mikroorganismus oder Esterase, erhältlich von einem Tier: 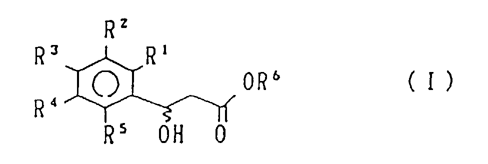 worin R¹, R², R³, R⁴ und R⁵ Wasserstoff, Hydroxyl, Alkoxy mit 1 bis 4 Kohlenstoffatomen, Benzyloxy, Fluor, Chlor oder Brom sind und R⁶ Alkyl ist, zur Bewirkung einer Umesterungsreaktion, und die Auflösung in ein optisch aktives Alkyl-3-aryl-hydroxypropionat, dargestellt durch die allgemeine Formel: 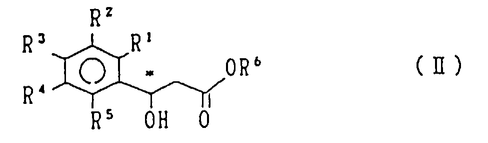 worin R¹, R², R³, R⁴, R⁵ und R⁶ die gleichen Bedeutungen wie oben beschrieben aufweisen, und ein optisch aktives Alkyl-3-aryl-3-acyloxypropionat umfaßt.

## Revendications

1. Procédé de production d'un 3-aryl-3-hydroxypropionate d'alkyle optiquement actif, caractérisé en ce que le procédé comprend les étapes consistant à faire réagir, en présence d'une estérase que l'on peut obtenir à partir d'un micro-organisme ou d'une estérase que l'on peut obtenir à partir d'un animal, un triglycéride avec un 3-aryl-3-hydroxy-propionate d'alkyle représenté par la formule générale : 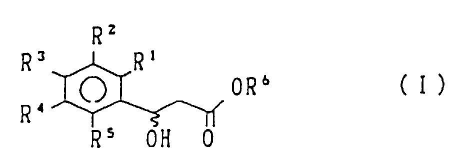 dans laquelle R¹, R², R³, R⁴ et R⁵ représentent un atome d'hydrogène, un groupe hydroxyle, un groupe alcoxy comportant 1 à 4 atomes de carbone, un groupe benzyloxy, un atome de fluor, de chlore ou de brome et R⁶ représente un groupe alkyle,
pour effectuer une réaction de transestérification, et à obtenir le dédoublement en un 3-aryl-3-hydroxypropionate d'alkyle optiquement actif représenté par la formule générale : 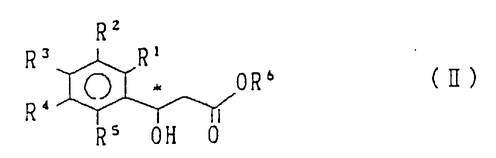 dans laquelle R¹, R², R³, R⁴, R⁵ et R⁶ ont les significations indiquées ci-dessus,
et un 3-aryl-3-acyloxypropionate d'alkyle optiquement actif.

2. Procédé de production d'un 3-aryl-3-hydroxypropionate d'alkyle optiquement actif, caractérisé en ce que le procédé comprend les étapes consistant à faire réagir, en présence d'une estérase que l'on peut obtenir à partir d'un micro-organisme ou d'une estérase que l'on peut obtenir à partir d'un animal, un ester vinylique d'acide gras et un 3-aryl-3-hydroxypropionate d'alkyle représenté par la formule générale : 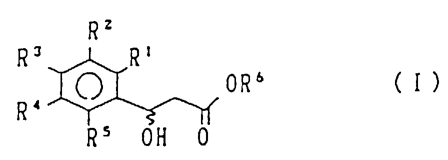 dans laquelle R¹, R², R³, R⁴ et R⁵ représentent un atome d'hydrogène, un groupe hydroxyle, un groupe alcoxy comportant 1 à 4 atomes de carbone, un groupe benzyloxy, un atome de fluor, de chlore ou de brome et R⁶ représente un groupe alkyle,
pour effectuer une réaction de transestérification, et à obtenir le dédoublement en un 3-aryl-3-hydroxypropionate d'alkyle optiquement actif représenté par la formule générale : 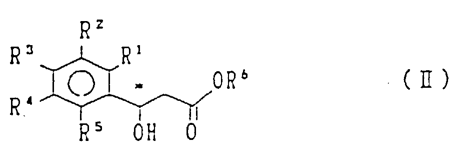 dans laquelle R¹, R², R³, R⁴, R⁵ et R⁶ ont les significations indiquées ci-dessus,
et un 3-aryl-3-acyloxypropionate d'alkyle optiquement actif.
